(19) Europäisches Patentamt
European Patent Office
Office européen des brevets

(11) EP 0 546 044 B1

## (12) EUROPEAN PATENT SPECIFICATION

(45) Date of publication and mention
of the grant of the patent:
**02.01.1997 Bulletin 1997/01**

(21) Application number: **91915873.3**

(22) Date of filing: **30.08.1991**

(51) Int Cl.6: **G01N 33/48**

(86) International application number:
**PCT/GB91/01477**

(87) International publication number:
**WO 92/04630 (19.03.1992 Gazette 1992/07)**

(54) **ANALYTICAL METHOD**

ANALYTISCHES VERFAHREN

PROCEDE D'ANALYSE

(84) Designated Contracting States:
**AT BE CH DE DK FR GR IT LI LU NL SE**

(30) Priority: **30.08.1990 GB 9018886
03.06.1991 GB 9111893**

(43) Date of publication of application:
**16.06.1993 Bulletin 1993/24**

(73) Proprietor: **UNIVERSITY COLLEGE OF WALES
ABERYSTWYTH
Aberystwyth, Dyfed SY23 3DD (GB)**

(72) Inventors:
• **KELL, Douglas, Bruce Symlog House
Aberystwyth Dyfed SY23 3HA (GB)**

• **WOODWARD, Andrew, Michael Calidris
Newborough
Gwynedd LL61 6RS (GB)**

(74) Representative: **Austin, Hedley William
Urquhart-Dykes & Lord
Alexandra House
1 Alexandra Road
Swansea West Glamorgan SA1 5ED (GB)**

(56) References cited:
**EP-A- 0 131 944        WO-A-89/08838
AU-B- 520 939**

## Description

The invention relates to a method of analysis of biological cell material, substrates therefor, or inhibitors of cell metabolism for the cell material.

The linear passive audio and radio frequency electrical properties of biological cell materials are well known. In the frequency range below about 10 MHz, these properties are conveniently measured as the equivalent parallel conductance and capacitance of an electrochemical cell containing the system under study. Up to these radio-frequencies, most biological cell materials exhibit two major dispersions, known as the alpha and beta dispersions. Whilst other sub-dispersions contribute to these major dispersions, and may occasionally be separated from them, the beta dispersion of tissues and cell suspensions is caused predominantly by the build-up of charge at the essentially non-conducting plasma membrane surfaces. The alpha dispersion, though not always dependent upon the ionic strength of the medium, is usually accounted for mainly in terms of the relaxation of counter-ions tangential to the charged surfaces of the membrane and cell envelope.

In the simplest case of dielectric relaxation, that of the reorientation of a "hard" sphere with a permanent dipole moment, the statistical mean of the cosine of the angle which the dipole makes with the field, has a field-dependence following the Langevin function:-

$$\cos <\theta> = \coth x - 1/x,$$

where $x = E/kT$. A Taylor expansion of this series shows that substantial deviations from linearity do not occur for values of $x$ less than approximately 1, and that to an excellent approximation $\cos <\theta> = \mu E/3kT$. Thus the dielectric displacement current is proportional to the magnitude of the exciting field, and their ratio, the admittance, independent of it. These properties are characteristic of a linear system obeying the fluctuation-dissipation therefrom.

Due to the fact that they are suspended or dissolved in conductive aqueous media, biological dielectrics are "lossy". Thus electrochemical reactions, and especially Joule heating, restrict the AC voltages that may be applied to them, and the dielectric properties of biological cell materials are typically measured using macroscopic electrical fields E of the order 0.1 to 5 V.cm$^{-1}$. Given the effective dipole moments usually encountered, the Langevin factor $\mu E/kT$ is normally minuscule, and, as judged by the independence of the measured admittance from the exciting field, well within the range of linearity.

It is known to measure the linear properties of biological materials using impedimetric instruments designed to filter out currents and voltages at frequencies other than the fundamental. This results in materials which appear to have linear characteristics but are in fact non-linear dielectrics.

We have surprisingly found that, by applying a pure sinusoidal AC current to excite a biological cell material, if instead of observing the AC current only at the frequency applied, the entire frequency range of interest is observed, a non-linear dielectric spectrum may be produced from a biological material at voltage levels at which hitherto, the material has been expected to exhibit linear properties only.

The entire frequency range of interest may be studied by performing a transformation on the signal, for example a Fourier transform.

According to a first aspect of the invention there is provided a method of analysing biological cell material, substrates therefor or inhibitors of cell metabolism for such cell material, said method comprising applying an AC electrical potential at one or more discrete frequencies to a sample of the bilogical material and determining a response at one or more frequencies which were substantially absent from, or not overlapping, with the applied AC potential.

When a field of appropriately low frequency is applied to a suspension of cells contained between two or more macroscopic electrodes, the charging of the membrane capacitance may cause a lesser but effective "amplification" of the macroscopic field across the membrane. In certain cases in which the membrane of interest contains appropriate enzymes this can cause performance of useful biological work in a field and frequency-dependent fashion. A general mechanism underlying this effect is that enzymes are not dipolar "billiard balls" and can relax between different conformations, some of which may and some of which may not have different vectorial dipole moments from each other.

The electrical potential applied to the sample of the biological material may comprise a potential field sufficient to excite the material and a low probing AC voltage to register the field-dependent dielectric properties of the biological material, which is lower than the applied potential field.

Preferably, however, a sinusoidal AC current is used to excite the material and the entire frequency range of interest is observed by performing a transformation to see the extent to which the non-linearities of the sample are manifest by the generation of harmonics. By varying the frequency and amplitude of the exciting current, a two-dimensional non-linear dielectric spectrum can be built up which can act as a dielectric fingerprint of the sample under test.

Advantageously a reference non-linear dielectric spectrum is produced using the supernatant of a biological material, the conductivity of which is adjusted to be substantially identical to that of the sample of the biological material. The spectrum from the sample is then divided by the reference spectrum, resulting in the deconvolution of the effects due to non linearities within the electrochemical system from those due to the biological cells themselves.

The third harmonic of the non-linear dielectric spectrum is of particular interest as its magnitude is indicative of the concentration of cells in the biological sample. The invention thus provides a method of observing the third harmonic in a non-linear dielectric spectrum obtained from a biological sample. It also provides a method of determining the concentration of cells in a cell suspension, since the magnitude of the third harmonic is indicative of the concentration of cells in such a suspension. The invention also provides a method of monitoring the ability of living cells to transduce exogenous electric field energy. The inventors have discovered that the particular harmonics present in the non-linear dielectric spectrum obtained from a biological sample are indicative of the metabolic state of living cells in a biological sample.

There is further provided by the present invention apparatus for carrying out a method as hereinbefore described, which apparatus comprises:-

a) retaining means for retaining a sample of living cell material;
b) means for applying an AC electrical potential across said sample so as to produce a non-linear dielectric spectrum; and
c) means for obtaining a detectable signal corresponding to said spectrum.

The production of power spectra using Fourier transformation results in the loss of phase information, and therefore only the magnitude of the 3rd harmonic (or indeed of any other harmonics) is used to display the energy transduced by the cells from the fundamental to the given harmonic in the present system. Thus the presence of negative harmonics is due simply to destructive interference between the biologically generated signal at a given harmonic and that from the external signal source. The cells do not "harvest" energy from the 3rd harmonic present in the exciting signal since no harmonics are observed when the fundamental voltage or frequency of the exciting signal are changed to those generated by our signal source when the fundamental is set as 2 V.cm$^{-1}$ and 20 Hz. Thus, variations in the magnitude of a harmonic may be caused by variations in its absolute magnitude and/or its phase relative to that of the 3rd harmonic present in the exciting signal. The data are presented in logarithmic (dB) scale, since this serves to accentuate the precision with which alterations in the observable magnitude of harmonics may be recorded.

Whilst there is some day-to-day variation in the exact magnitude of the 3rd harmonic (when viewed on the logarithmic decibel scale), the data obtained are very reproducible within a few dB, particularly for a given batch of cells. Similarly, using identical "samples" (or "reference" supernatants) in both electrochemical cells, no harmonics are observed, (i) indicating that the surface electrochemistry of their electrodes is well-matched, and (ii) that living cells are indeed the source of the 3rd harmonic observed.

The electrical fields involved in the following experiments are (in terms of the Langevin factor) quite minuscule, and the system would normally be expected to lie well within the domain of linearity. Indeed, observations of the fundamental, and linear impedance measurements using a Hewlett-Packard 4192A Impedance Analyser indicated that the linear impedance was independent (within experimental error) of the magnitude of the exciting voltage in the range studied. Thus, it is quite feasible to have a system in which the impedance appears linear but which is in fact nonlinear. The potential-dependant capacitance (thickness) of a bilayer membrane can exhibit a quadratic dependence upon the potential difference across it. However, at the potentials used here the quadratic term is negligible.

Membrane proteins are particularly powerful candidates for interacting with electrical fields for a variety of reasons, including the following: (i) the membrane protein cannot rotate from one side of the membrane to the other and dissipate electrical energy by simple Debye-like rotation of this type; (ii) as described above, the membrane can "amplify" the exciting signal; (iii) membrane proteins have substantial dipole moments. In addition, of course, in common with all proteins, they can effect transitions between different conformational states possessing different dipole moments. Thus in seeking a mechanistic basis for the remarkable generation of nonlinear dielectricity that we have observed one is led to consider the membrane properties of this organism.

A washed cell suspension of S. cerevisiae with no added carbon substrate, slowly metabolising endogenous stores under the presumably essentially anaerobic conditions pertaining in the relatively concentrated suspensions normally used, may be expected to generate ATP by substrate-level phosphorylation. As a major potential mechanism of "slip" or "overflow metabolism", this ATP may be expected to be utilised by the H$^+$-ATPase present in this organism to drive the uptake of cations such as K$^+$ from the external medium until a state of "static head" is attained, in which the free energy of hydrolysis of ATP is balanced against the free energy stored in the K$^+$ gradient (and indeed those of other ions). The distribution of enzymic conformational macrostates would then be approximately determined by their basic free energies and the net turnover (rate of entropy production) of the enzyme would be minimal. In this sense the energetic macrostates of the enzyme may then be regarded as a symmetrical potential well. This would then explain the generation of odd but not even harmonics. We have determined that the H$^+$-ATPase in the plasma membrane of this organism, as the main enzyme potentially active under the physiological conditions used, is the major source of the nonlinear dielectric response observed.

The inventors have shown for the first time in cell suspensions that the conformational flexibility of enzymes can manifest in the generation of nonlinear dielectric spectra at very modest values of the exciting field, in which the Langevin factor is extremely small and in which purely linear behaviour would formerly be expected.

The invention will now be described by way of example only, with reference to the accompanying drawings, in which:

Figure 1 is a schematic representation of a non-linear spectrometer for carrying out the method according to the invention;
Figure 2 is a schematic representation of the electrochemical cell used in the spectrometer of Figure 1;
Figure 3 is a block diagram of a particular method used to obtain non-linear dielectric spectra uncontaminated by artefactual electrochemical phenomena;
Figure 4 is a representation of a non-linear dielectric spectrum of Saccharomyces cerevisiae (S. cerevisiae);
Figure 5 shows a non-linear dielectric spectrum obtained from a reference cell;
Figure 6 shows a spectrum obtained by subtracting the spectrum of Figure 4 from the spectrum of Figure 5;
Figure 7 is a non-linear dielectric spectrum obtained from a suspension of boiled cells;
Figure 8 (a) - (c) show the effect of averaging on the non linear dielectric spectrum of S. cerevisiae;
Figure 9 shows the effect of cell concentration on the magnitude of the third harmonic in the non-linear dielectric spectrum of S. cerevisiae;
Figure 10 shows the effect of exciting frequency on the magnitude of the third harmonic of the nonlinear dielectric spectrum of S. cerevisiae;
Figure 11 shows the effect of the strength of the exciting field on the magnitude of the 3rd harmonic of the non-linear dielectric of S. cerevisiae;
Figure 12 shows the effect of sodium metavanadate on the magnitude of the third harmonic of the nonlinear dielectric spectrum of S. cerevisiae;
Figure 13 shows the effect of glucose on the non-linear dielectric spectrum of S. cerevisiae;
Figure 14 shows another embodiment of a non-linear dielectric spectrum of S. cerevisiae cells;
Figure 15 shows a non-linear dielectric spectrum of an anaerobic suspension of Micrococcus luteus; and
Figure 16 shows a non-linear dielectric spectrum of an aerobic suspension of Micrococcus luteus.

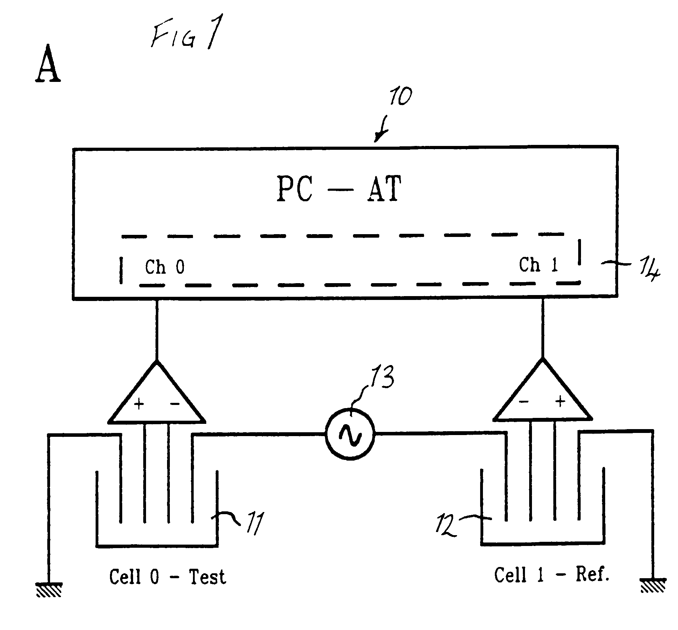

Referring to Figure 1, a nonlinear dielectric spectrometer for use in carrying out the method according to the invention is designated generally by the reference numeral 10. The spectrometer 10 comprises two electrochemical cells 11,12. Cell 11 is a test cell containing a sample of the biological system whose nonlinear dielectric properties are being studied. In this example the biological system comprises a system comprising a suspension of S. Cerevisiae. The concentration of the cells was approximately 50mg dry wt. ml$^{-1}$ in a medium of 20 mM $KH_2PO_4$, 30 mM KCl, 1mM $MgCl_2$ pH 6.5. The tests were carried out within four hours of preparing the suspension. Cell 12 is a reference cell comprising the supernatant of the test suspension, the conductivity of which had been adjusted with distilled water, to compensate for the volume fraction of cells present in the sample to be identical to that of the sample at the frequency of interest. The cells 11,12 are connected to a sinusoidal oscillator 13, via a Data Translation Analog - to Digital converter, to an 80386 - based microcomputer 14.

The system is built around an IBM-PC-AT compatible microcomputer (Viglen III, 80386 main processor from Viglen Ltd, LONDON U.K.). To maximise the performance of the mathematical operations, an 80387 compatible co-processor (Cyrix 83C87) was used. One of the expansion ports of the computer was furnished with a Data Translation model 2823 ADC/DAC board. Because of the expectation that the harmonic signals would have a low magnitude, a 16-bit board was chosen with 4 differential inputs and a stated upper frequency of 100 kilosamples/second.

Referring to Figure 2, an electrochemical cell 21 is shown. To minimise the contribution of electrode polarisation phenomena a 4-electrode system based on gold pin-type electrodes 22 was used. Signals were applied to the outer, current electrodes by means of a Thandor TG501 function generator (RS Components Ltd). The frequency and amplitude of these signals were checked using a Solartron 1200 Signal Processor (Schlumberger Instruments, Farnborough, Hants) and a Hameg HM 208 Digital Storage Oscilloscope.

The acquisition of the data and its subsequent processing and display was performed using the ILS software (Signal Technology Inc, Geoleta, California) running under control files.

The spectra were collected using the following method:
the sample cell suspension was pipetted into the electrode cell 11 (Fig 1), the test waveform applied to the (outer) current electrodes, and the data logged from the (inner) voltage electrodes at a sampling frequency (which was typically at 25 times the frequency of the fundamental) and for a time specified by the operator. Time was specified in terms of a number of blocks, each block consisting of 512 samples. At the end of the time specified, the data were Fourier transformed as follows. Preliminary pre-whitening was carried out by subtracting the mean of the block from the individual samples. Each block was then windowed using a Blackman window and fast-Fourier-transformed using routines within the ILS software to form an ensemble of power spectra. These were then averaged in order to enhance the signal:noise ratio. The spectral data were stored on the computer's hard disk.

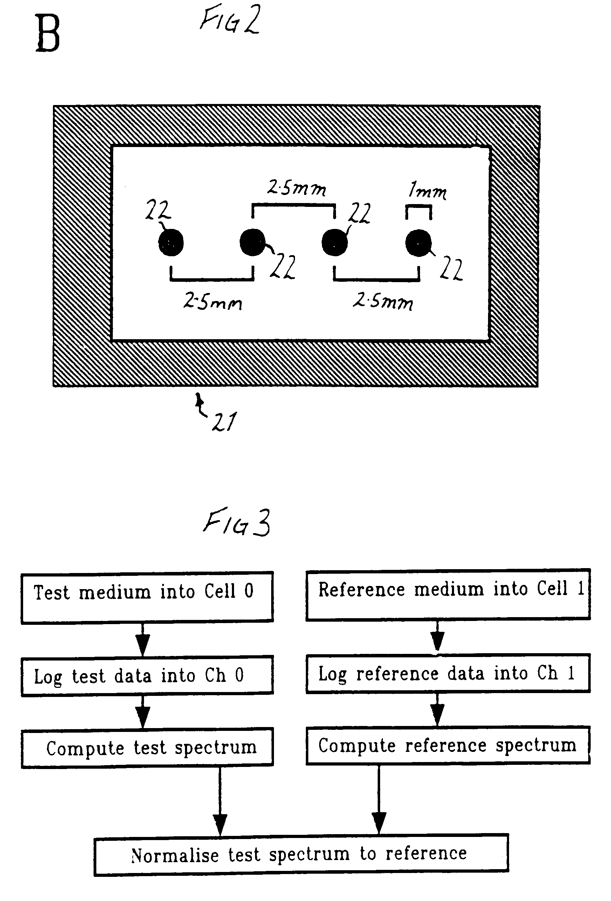

A reference spectrum was acquired using the supernatant in cell 12 (Fig 1), whose conductivity had been adjusted (with distilled water), to compensate for the volume fraction of cells present in the sample to be identical to that of the sample at the frequency of interest. Two different types of control file were used, depending upon whether the reference was to be logged using the same set of electrodes or (as was done in the experiments described herein) a separate matched cell. In either case, the logging, windowing and Fourier transformation routines were identical, and provided a power spectrum of the "reference" cell, which was also logged on the computer's hard disk. Finally the "sample" power spectrum so obtained was divided by the "reference" power spectrum, and also stored on the disk. The total time necessary to acquire a difference dielectric spectrum (at 20 Hz, 500 samples per second for 10 blocks) was some 2.5 minutes. A diagram of the steps involved in the generation of the non-linear dielectric spectra is shown in Fig 3. The advantage of this approach was that it allowed the effects due to non-linearities within the electrochemical system from these due to the biological cells themselves to be deconvolved.

The spectrometer 10 may be used for the registration of non-linear biological dielectric spectra, to indicate for the first time that they are easily observed in cell suspensions, and to show that the $H^+$ - ATPase in the plasma membrane of *Saccharomyces cerevisiae* is the source of the majority of the non-linear dielectric response thus observed.

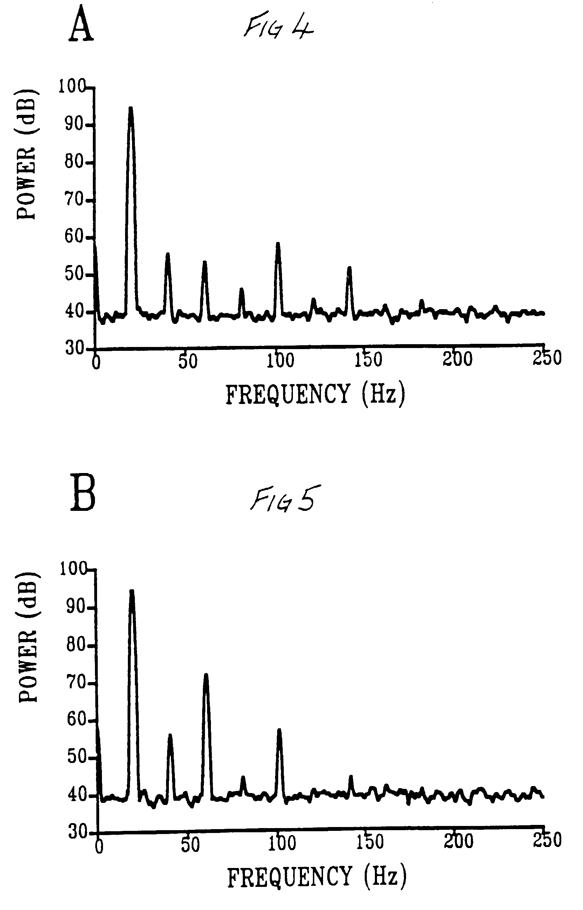

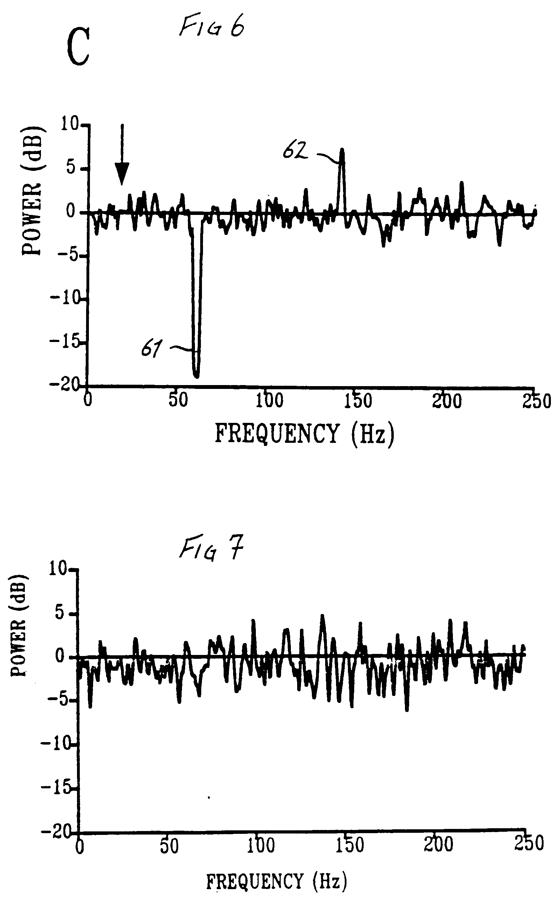

Figure 4 shows a typical non-linear dielectric spectrum obtained from a suspension of resting cells of *S. cerevisiae* using the spectrometer of Figure 1, with an exciting voltage (measured between the outer electrodes) of 1.5V (2.0 V.cm$^{-1}$) at a frequency of 20 Hz, and displays spectra from the sample (Figure 4) the reference (Figure 5) and their difference (Figure 6). Due to imperfections in the generator and the nonlinearities inherent in electrochemical systems, the applied waveform is not purely sinusoidal but contains harmonic components which, although very small by comparison with the energy in the fundamental, may yet be observed using a measuring system with the present (16-bit) sensitivity and a logarithmic display. The pattern of harmonics between the "sample" and "reference" cells is markedly different, and upon subtraction of the reference spectrum from the sample spectrum a very strong and apparently negative third harmonic 61 (Fig 6) is obtained. A (positive) 7th harmonic 62 is also reproducibly observed (and on occasion an 11th harmonic), but even harmonics are absent under the stated conditions.

Because of the use of the reference spectrum method, it was clear that the generation of a 3rd harmonic depended upon the presence of yeast cells. Figure 7 shows that no third harmonic is generated using dead (boiled) cells. This indicated that the presence of potentially active enzymes was a prerequisite to the generation of a third harmonic.

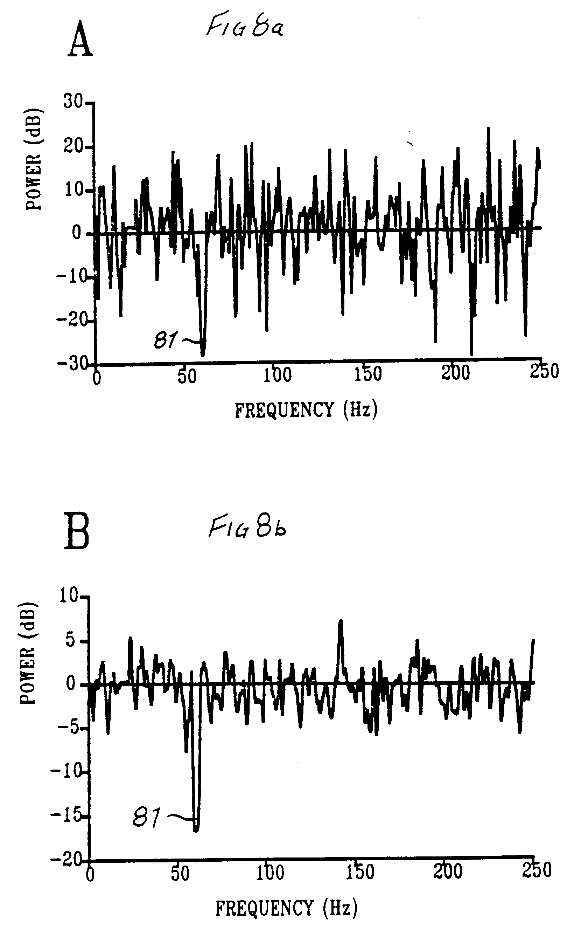

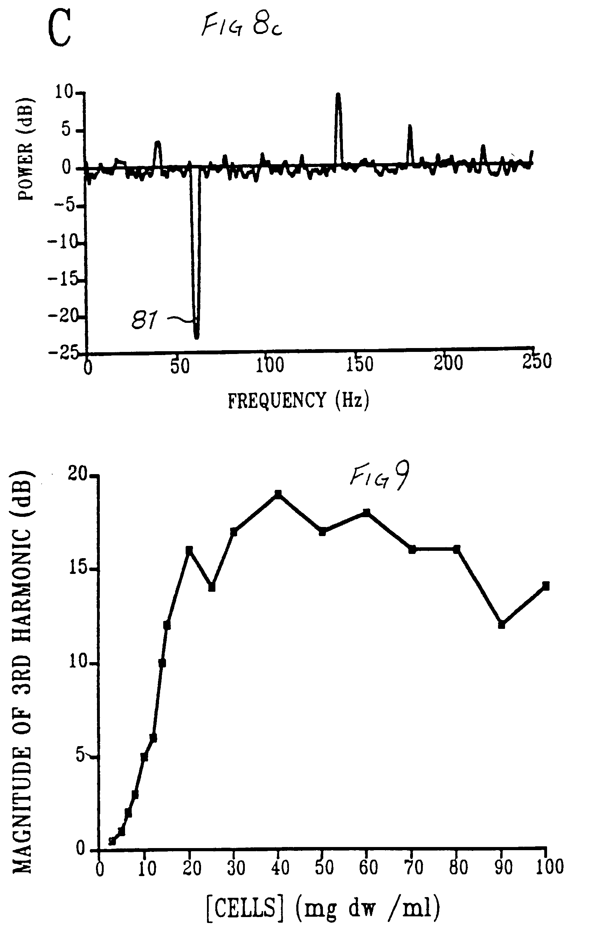

To establish whether only harmonics were generated, or whether the power spectra contained non-harmonic components, the number of blocks that were averaged was varied. The data from a representative set of runs are displayed in Figs 8a to 8c where it may be observed that the magnitude of the 3rd harmonic 81 remains essentially constant in the face of a highly variable degree of noise, the variance of the noise decreasing (as expected) in proportion to the number of blocks, such that no true non-harmonic components could be discerned.

The dependence of the magnitude of the 3rd harmonic as a function of the concentration of cells is shown in Fig 9 where it may be observed that the magnitude of the 3rd harmonic in dB is substantially linear with the concentration of cells up to a cell concentration of some 25 mg dry wt.ml$^{-1}$ whereupon a transition to a plateau region may be observed.

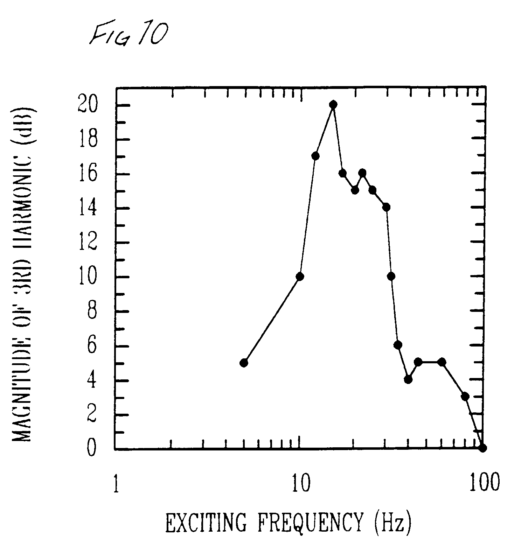

The 3rd harmonic generated was usually maximal when the exciting frequency was some 15-20 Hz. Fig 10 displays the magnitude of the 3rd harmonic as a function of the exciting frequency. It may be observed that as the frequency is increased above or decreased below some 15-20 Hz, the magnitude of the 3rd harmonic drops off rather sharply.

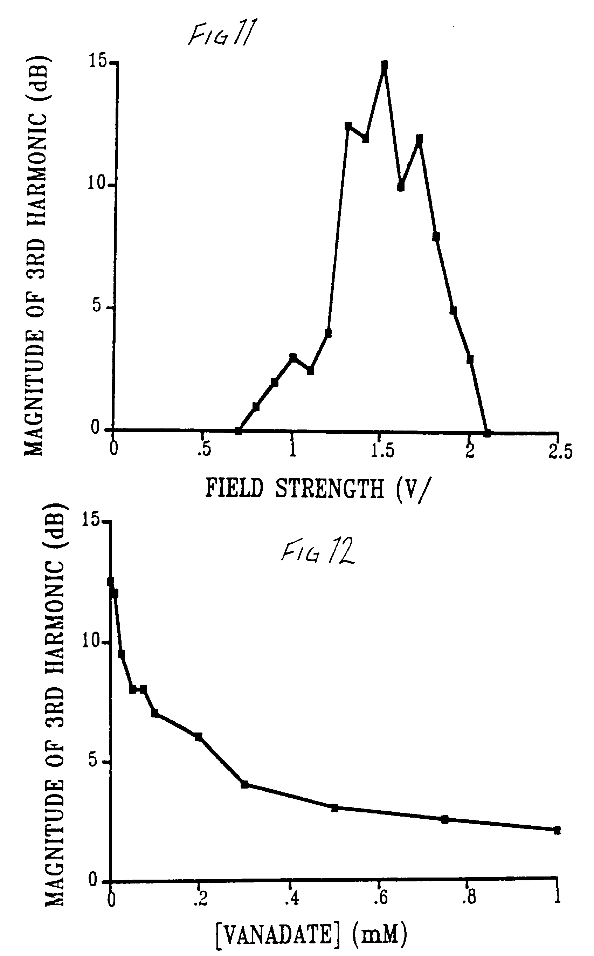

As well as the above frequency window, there was an even sharper voltage or amplitude window within which nonlinear dielectric behaviour could be observed. Fig 11 shows that the magnitude of the 3rd harmonic is only significant in a voltage window between about 0.6 and 2.1 V (0.8 - 2.8 V.cm$^{-1}$). When the exciting frequency was varied, the amplitude window observed did not appear to change significantly (data not shown). If the measurement was carried out in the presence of an additional electrostatic (DC) field, the magnitude of the 3rd harmonic was strongly decreased, disappearing completely when the DC field exceeded 0.4 V.cm$^{-1}$ (and the exciting AC field was 2.0 V.cm$^{-1}$).

It is well known that the catalytic cycle of enzymes of this type (the so-called $E_1E_2$ enzymes) involves an enzyme-bound phosphate intermediate, and that their activity can be inhibited by low concentrations of pentavalent vanadium compounds whose trigonal bipyramidal structure is thought to mimic the transition state of the phosphate during its hydrolysis, trapping the enzyme in its $E_2$ conformation.

Figure 12 shows the effect of quite modest concentrations of vanadate on the magnitude of the 3rd harmonic, where it may be observed that the generation of this harmonic is essentially completely abolished by 1 mM sodium metavanadate, and with a $K_i^{app}$ (when the ordinate is plotted using a Db scale) of approximately 0.15 Mm. This again suggests strongly that the $H^+$-ATPase in the plasma membrane of these cells is the main source of the non-linear dielectric response, and further serves to leave phenomena such as dielectrophoresis as the source of the non-linearities observed. The harmonics were also completely abolished by the $H^+$-ATPase inhibitor dibenzhydryl carbodiimide at a concentration of 0.2 pmol/mg dry wt of cells.

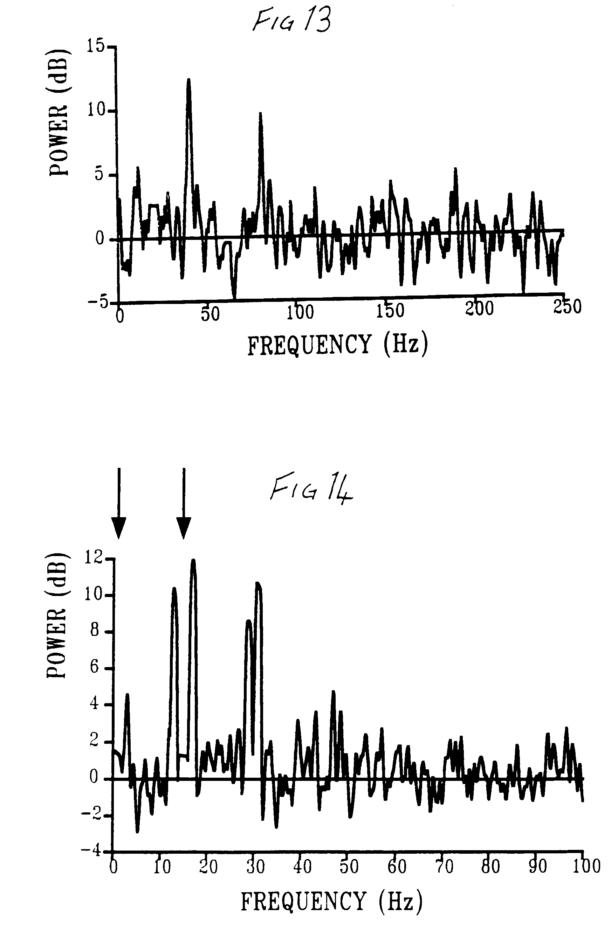

As described above, a 3rd harmonic was reproducibly observed with resting cell suspensions of S. cerevisiae. This could be ascribed to the presence of the H⁺-ATPase in this organism, and should be expected to reflect a situation in which the enzyme was at static head. An experiment to determine how this behaviour might be modified when the enzyme was driven away from static head and was able (or expected) to do work was carried out. Resting cells were taken, their (usual) non-linear dielectric spectrum recorded, and a metabolisable carbon source (D-glucose) added. After a short lag period of some 20 minutes, the spectrum displayed in Fig 13 was recorded. Remarkably, the 3rd harmonic had disappeared and was replaced by substantial 2nd and 4th harmonics. These even harmonics were also vanadate-sensitive. When static head was again attained, the spectrum, returned to its starting shape, with a substantial 3rd but no even harmonics. This behaviour is consistent with the view that when carrying out net work (energy transduction), the enzyme represents an asymmetric potential well with the rectification necessary for the adsorption of exogenous electric field energy. A parallel measurement of the dielectric permitivity at 0.3 Mhz, a monitor of intact cellular biomass, did not show any observable changes during this experiment. Thus non-linear dielectric spectroscopy provides a sensitive means of distinguishing the metabolic states of living cells.

Non-linear dielectric properties may also be manifested as the frequency mixing of a plurality of fundamentals. Fig 14 shows the non-linear dielectric spectrum recorded when a suspension of S. cerevisiae was excited with fields (0.9V/cm each) at frequencies of 1Hz and 15 Hz. The frequency mixing to produce signals at sums and differences of integral multiples of the fundamental is clear.

The electric potential Vm generated across the (spherical) yeast plasma membrane is given by Vm = 1.5 Re cos where E is the microscopic field, r the cell radius and the angle between the field and the membrane normal.

For the present cell radius of 3 microns and a field of 2 V.cm⁻¹ a maximum (field-dependent change in) membrane potential of 0.9mV was obtained. For a membrane thickness of 5nm, the maximum oscillatory trans-membrane field $E_m$ is 1800 V.cm⁻¹ Typical membrane proteins have permanent dipole moments m of 100-1000 Debye units. For convenience we will assume that relevant changes in dipole moment due to field-dependent conformational changes of target enzymes are 500D (they are however likely to be much less since 500D equates to the displacement of 10 full charges across the membrane). The Langevin factor $\mu E/kT$ is then equal to some 0.075, i.e. substantially less than 1. Thus despite the application of a field which would normally be regarded as very modest, we have observed the generation of a substantial third harmonic by the S. cerevisiae cells.

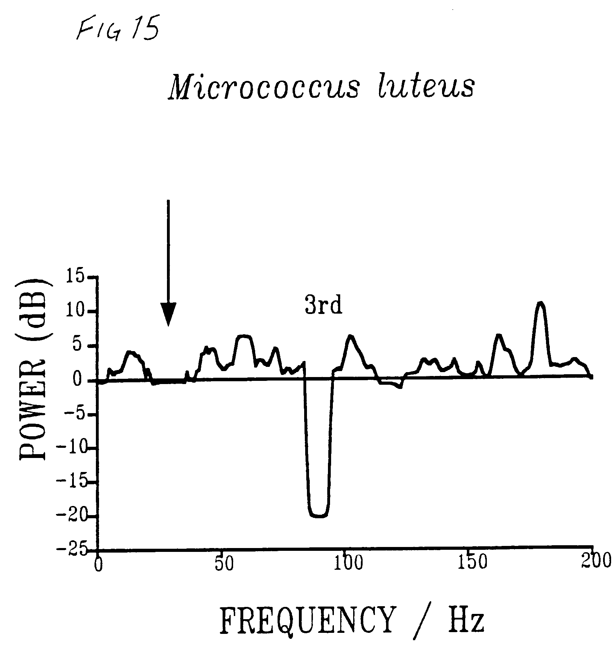

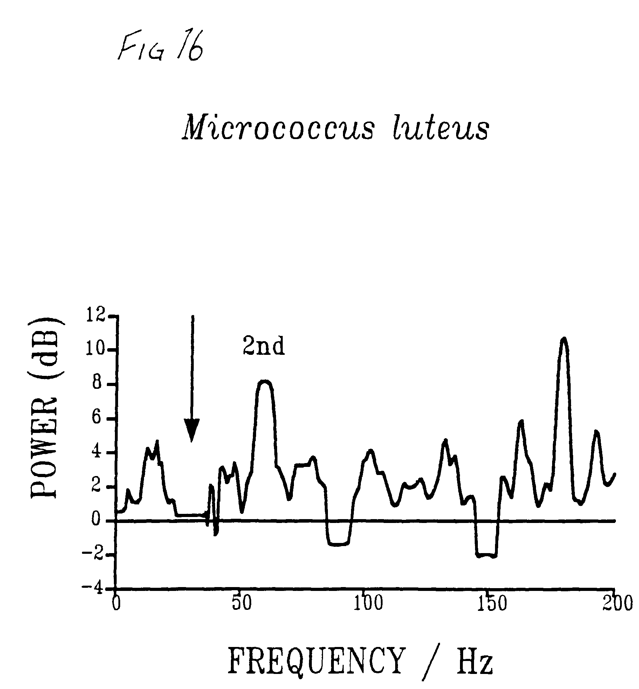

The invention as hereinbefore described has been carried out using suspensions of S. cerevisiae, with or without glucose; however the method is of general applicability and is not limited to the use of the former substrates. Figure 15 shows a spectrum illustrating the non-linear dielectric behaviour of an anaerobic suspension of Micrococcus luteus. Since this organism cannot ferment, such cells are resting, and display an odd-numbered (third) harmonic. Figure 16 shows an aerobic suspension of Micrococcus luteus where the organism can respire and which causes the generation of an even-numbered (second) harmonic.

Substrates which can be determined by the method according to the invention include oxygen, glucose, lactic acid or lactate, or the like. Similarly, inhibitors of cell metabolism can be determined by the method according to the invention; examples of such inhibitors include vanadates, as described above with reference to Figure 12.

## Claims

1. A method of analysing biological cell material, substrates therefor, or inhibitors of cell metabolism for said cell material, said method comprising applying an AC electrical potential at one or more discrete frequencies to a sample of the biological material, and determining a response at one or more frequencies which were substantially absent from, or not overlapping with, the applied AC potential.

2. A method according to claim 1, wherein the electrical potential comprises a potential field sufficient to excite the system, and a probing AC voltage sufficient to register the field-dependent dielectric properties of the biological material, wherein the probing voltage is lower than the electric potential applied to excite the system.

3. A method according to any of claims 1 or 2, wherein said potential is sinusoidal, and the entire frequency range of interest is observed by performing a translation to ascertain the extent to which the non-linearities of the material are manifest by the generation of harmonics.

4. A method according to any of claims 1 to 3, wherein said material comprises a cell suspension.

5. A method according to claim 4, wherein an AC electric potential at one or more discrete frequencies is applied to a supernatant of said dispersion or suspension, the conductivity of said supernatant being adjusted to be substantially identical to that of said biological material.

6. A method according to any of claims 1 to 5, which further comprises observing the third harmonic of said response.

7. A method according to any of claims 1 to 6, which further comprises observing even-numbered harmonics of said response.

8. Apparatus for analysing biological cell material, substrates therefor, or inhibitors of cell metabolism for said cell material, which apparatus comprises:

a) retaining means for retaining a sample of said biological material;
b) means for applying an AC electrical potential at one or more discrete frequencies to said sample; and
c) means for determining a response at one or more frequencies which were substantially absent from or not overlapping with the applied AC potential.

**Patentansprüche**

1. Verfahren zum Analysieren von biologischem Zellmaterial, Substraten hierfür oder von Inhibitoren des Zellstoffwechsels für das genannte Zellmaterial, wobei das Verfahren das Anlegen eines elektrischen Wechselpotentials bei einer oder mehreren diskreten Frequenzen an eine Probe eines biologischen Materials und das Bestimmen einer Antwort bei einer oder mehreren Frequenzen, die im wesentlichen nicht bei der angelegten Wechselspannung liegen und nicht damit überlappen, beinhaltet.

2. Verfahren nach Anspruch 1, worin das elektrische Potential ein Potentialfeld umfaßt, das ausreicht, das System anzuregen, und eine Untersuchungswechselspannung umfaßt, die ausreicht, die feldabhängigen dielektrischen Eigenschaften des biologischen Materials zu registrieren, wobei die Untersuchungsspannung niedriger ist als das zum Anregen des Systems angelegte elektrische Potential.

3. Verfahren nach Anspruch 1 oder 2, worin das Potential sinusförmig ist und der gesamte interessierende Frequenzbereich durch das Ausführen einer Übertragung beobachtet wird, um das Ausmaß zu ermitteln, bis zu dem die Nichtlinearitäten des Materials aufgrund der Erzeugung von Oberwellen deutlich sind.

4. Verfahren nach einem der Ansprüche 1 bis 3, worin das genannte Material eine Zellsuspension umfaßt.

5. Verfahren nach Anspruch 4, worin ein elektrisches Wechselpotential bei einer oder mehreren diskreten Frequenzen an einen Überstand der genannten Dispersion oder Suspension angelegt wird, wobei die Leitfähigkeit des Überstands derart eingestellt wird, daß sie im wesentlichen mit jener des biologischen Materials identisch ist.

6. Verfahren nach einem der Ansprüche 1 bis 5, das weiterhin das Beobachten der dritten Oberwelle der genannten Antwort umfaßt.

7. Verfahren nach einem der Ansprüche 1 bis 6, das ferner das Beobachten der geradzahligen Oberwellen der genannten Antwort umfaßt.

8. Vorrichtung zum Analysieren von biologischem Zellmaterial, Substraten hierfür oder Inhibitoren des Zellstoffwechsels für das Zellmaterial, wobei die Vorrichtung folgendes aufweist:

a) Ein Rückhaltemittel zum Zurückhalten einer Probe des genannten biologischen Materials;

b) ein Mittel zum Anlegen eines elektrischen Wechselpotentials bei einer oder mehreren diskreten Frequenzen an die genannte Probe; und

c) ein Mittel zum Bestimmen einer Antwort bei einer oder mehreren Frequenzen, die im wesentlichen bei dem angelegten Wechselpotential nicht vorhanden sind und damit nicht überlappen.

**Revendications**

1. Procédé pour analyser un matériau biologique cellulaire, des substrats pour celui-ci, ou des inhibiteurs du métabolisme cellulaire dudit matériau cellulaire, ledit procédé comprenant l'application d'un potentiel électrique du courant alternatif à une ou plusieurs fréquences discrètes à un échantillon du matériau biologique, et pour déterminer une réponse à une ou plusieurs fréquences qui étaient sensiblement absentes du potentiel du courant alternatif appliqué, ou qui ne le chevauchaient pas.

2. Procédé selon la revendication 1, dans lequel le potentiel électrique comprend un champ de potentiel suffisant pour exciter le système, et une tension du courant alternatif de sonde suffisante pour enregistrer les propriétés diélectriques dépendantes du champ du matériau biologique, dans lequel la tension de sonde est inférieure au potentiel électrique appliqué pour exciter le système.

3. Procédé selon l'une quelconque des revendications 1 ou 2, dans lequel ledit potentiel est sinusoïdal, et toute la gamme de fréquence concernée est observée en réalisant une translation pour vérifier le degré des non linéarités dans le matériau par la génération d'harmoniques.

4. Procédé selon l'une quelconque des revendications 1 à 3, dans lequel ledit matériau comprend une suspension cellulaire.

5. Procédé selon la revendication 4, dans lequel un potentiel électrique du courant alternatif à une ou plusieurs fréquences discrètes est appliqué à un surnageant de ladite dispersion en suspension, la conductivité dudit surnageant étant ajustée de manière à être sensiblement identique à celle dudit matériau biologique.

6. Procédé selon l'une quelconque des revendications 1 à 5, qui comprend en outre l'observation du troisième harmonique de ladite réponse.

7. Procédé selon l'une quelconque des revendications 1 à 6, qui comprend en outre l'observation d'un nombre entier d'harmoniques de ladite réponse.

8. Application pour analyser un matériau biologique cellulaire, un substrat pour celui-ci, ou des inhibiteurs du métabolisme cellulaire dudit matériau cellulaire, ledit appareil comprenant :

a) des moyens de retenue pour retenir un échantillon dudit matériau biologique ;
b) des moyens pour appliquer un potentiel électrique du courant alternatif à une ou plusieurs fréquences discrètes audit échantillon ; et
c) des moyens pour déterminer une réponse à une ou plusieurs fréquences qui étaient sensiblement absentes du potentiel du courant alternatif appliqué, ou qui ne le chevauchaient pas.

A

FIG 1

B

FIG 2

2·5mm

1mm

22

22

22

22

22

2·5mm

2·5mm

21

FIG 3

| Test medium into Cell 0 | Reference medium into Cell 1 |
|---|---|
| Log test data into Ch 0 | Log reference data into Ch 1 |
| Compute test spectrum | Compute reference spectrum |

Normalise test spectrum to reference

A  FIG 4

B  FIG 5

C    FIG 6

FIG 7

A    FIG 8a

B    FIG 8b

C   FIG 8c

POWER (dB)

81

FREQUENCY (Hz)

FIG 9

MAGNITUDE OF 3RD HARMONIC (dB)

[CELLS] (mg dw /ml)

Fig 10

FIG 11

MAGNITUDE OF 3RD HARMONIC (dB)

FIELD STRENGTH (V/

FIG 12

MAGNITUDE OF 3RD HARMONIC (dB)

[VANADATE] (mM)

Fig 13

Fig 14

FIG 15

*Micrococcus luteus*

FIG 16

*Micrococcus luteus*